# EUROPEAN PATENT APPLICATION

(11) **EP 1 790 316 A1**
(43) Veröffentlichungstag der Anmeldung: **30.05.2007**
(21) Anmeldenummer: 05763347.1
(22) Anmeldetag: 10.06.2005
(51) Int. Cl.: A61F 2/14, A61L 27/56, A61L 27/16

(54) **IMPLANTAT ZUR VERSTÄRKUNG DER HORNHAUT UND KERATOPROTHESE MIT EINER PLATTE AUS PORÖSEM POLYTETRAFLUORETHYLEN**

(30) Priorität: 17.06.2004 RU 2004119479; 17.06.2004 RU 2004119480
(71) Anmelder: ZAKRYTOE AKCIONERNOE OBSCHESTVO"NAUCHNO- PROIZVODSTVENNY COMPLEX "ECOFLON", St.Petersburg 191040 (RU)
(72) Erfinder: DYAKOV, Valery Evgenievich, St.Petersburg, 194356 (RU); ASTAHOV, Yury Sergeevich, St.Petersburg, 190031 (RU); BOYKO, Ernest Vitalievich, St.Petersburg, 191028 (RU); USHAKOV, Nikolai Andreevich, St.Petersburg, 194175 (RU); HAPCHAEV, Ruslan Talasovich, St.Petersburg, 193171 (RU); KORTUNOV, Yury Anatolievich, St.Petersburg 193171 (RU); FEDOTOVA, Lubov Mihailovna, St.Petersburg, 195176 (RU)
(74) Vertreter: Jeck, Anton
(86) Internationale Anmeldenummer: PCT/RU2005/000334
(87) Internationale Veröffentlichungsnummer: WO 2006/009490

(57) **Zusammenfassung**

Das Implantat gemäß der Erfindung zur Verstärkung der Augenhornhaut ist aus porösem Polytetrafluorethylen hergestellt, dessen Aufbau in Form von Polymerelementen und Hohlräumen mit Verbindungen dieser Hohlräume in ein dreidimensionales Netz verkörpert ist, das einen Hohlraumanteil von 15-40 %, eine spezifische Holraumoberfläche von 0,25-0,55 mkm²/mkm³, einen Durchschnittsabstand zwischen den Hohlräumen von 25-50 mkm und eine durchschnittliche Strangstärke von 8-25 mkm aufweist. Dieses Implantat ist in Form einer Konvexkonkavlinse verkörpert, dessen Krümmungsradius 7,0-10,0 mm beträgt. Die Keratoprothese gemäß der Erfindung weist ein optisches Element aus optisch transparentem Material und eine mit diesem Element verbundene Tragplatte aus porösem, den genannten Aufbau aufweisenden Polytetrafluorethylen auf. Das optische Element der Keratoprothese kann abnehmbar ausgebildet sein.

## Beschreibung

Die Erfindung gehört zum medizinischen Gebiet, nämlich zur Ophtalmologie, und zwar zu implantierbaren Augenprothesen.

Trübungen der Hornhaut (der Cornea) entstehen infolge penetrierender Kornealskleralwunden, Verbrennungen und Verätzungen des Auges sowie Hornhautdystrophie usw. Grobe Gefäßleukome und Retrokornealschwarten führen zum vollständigen Verlust der Sehkraft. Eine Transplantation der Hornhaut (Keratoplastik) stellt sich für die vaskulierten, groben Leukome und ödematöse Hornhautdystrophie als unwirksam heraus. Die Keratoprothetik ist die einzige Möglichkeit, dem Kranken eine minimale Sehkraft zu geben.

Die moderne Keratoprothese umfasst in der Regel ein optisches Element aus optisch transparentem Material in Form eines Zylinders, einer Linse usw. und eine mit ihm verbundene Tragplatte in Form eines perforierten Rings, eines Rings mit Nadeln, eines Ohrs bzw. einer amöbenähnlichen Form. Unterschiedliche Arten und Formen einer Keratoprothese sind im Buch von S. N. Fjodorow (S. N. Fjodorow, S. I. Moros, W. K. Sujew, Keratoprothetik, Moskau, Verlag Medizin, 1982, S. 5-53) beschrieben. Während der Operation wird das Tragteil der Keratoprothese in die intralamelläre Hornhauttasche eingeführt.

Eine häufige, spezifische Komplikation der Keratoplastik, die zur Abstoßung der Keratoprothese führt (vgl. d. S. 97-113), ist die aseptische Hornhautnekrose vorne an der Tragplatte. Eine der Entstehungsursachen aseptischer Nekrosen ist die nicht ausreichende Versorgung der Hornhautschichten, die über der Tragplatte liegen, mit lebensnotwendigen Stoffen. Zur Vorbeugung bzw. Beseitigung dieser Komplikation werden Hilfsoperationen durchgeführt, um die Hornhaut (Leukom) mit einem passenden Implantat zu stärken. Bekannt ist ein Autotransplantat, beispielsweise die Schleimhaut der Lippen, das auf das Leukom verpflanzt wird (N. A. Putschkowskaja et al., Ophtalmologische Zeitschrift, 1978, S. 498-500, Nr. 7). Außer der autologen Transplantation der Schleimhaut wird dem Kranken eine intralamelläre Kerato- bzw. Skleraplastik mit Hilfe der mit Silikagel getrockneten bzw. frischen, allogenen Hornhaut- oder Skleraltransplantate nach 1,5-2 Monaten eingesetzt. Die Keratoprothetik wird 3-4 Monate nach der Leukomstärkung durchgeführt.

Im Laufe dieser Operation aber wurde eine Verdünnung des transplantierten, autologen Materials, wie Lippenschleimhaut, und die beinahe vollständige Resorption (Lysis) von Allotransplantaten beobachtet. Die Skleraltransplantate wurden weniger lysiert als die Allotransplantate der Hornhaut, waren aber nur locker mit den Gefäßen verwachsen, und es konnte deren weitere Lysis erwartet werden.

Bekannt ist auch ein autogenes Implantat zur Hornhautstärkung, wie Knorpel der Ohrmuschel (M. M. Krasnow et al., Ophtalmologische Zeitschrift, 1978, Nr. 7, S. 392-394). Der Knorpel ist ziemlich elastisch, ausreichend dicht und dem Hornhautgewebe histologisch ähnlich. Zur vollständigen Implantateinheilung sind 3-4 Monate ausreichend, und danach kann die Keratoplastik durchgeführt werden.

Bekannt ist ein autogenes Implantat, wie die Knochenhaut des Schienbeins (W. W. Wolkow, N. A. Uschakow, Fragen der wiederherstellenden Opthalmologie, Leningrad, 1972, S. 37). Ein Teilchen der Knochenhaut wird in die Tasche des in mittleren Schichten befindlichen Leukoms eingeführt und mit einer Knotennaht an tiefere Leukomschichten befestigt. Die Knochenhaut heilt in der Regel in den Leukomschichten gut ein. Die Keratoprothetik ist nach 2-3 Monaten möglich, wobei die Tragplatte unter der Schicht der eingeheilten Knochenhaut angeordnet wird.

Ein allgemeiner Nachteil der Autotransplantate ist deren Lysisanfälligkeit in früherer oder späterer, postoperativer Zeit, wodurch oft wiederholte, operative Eingriffe erforderlich sind. Ferner ist die Entnahme von Autotransplantaten mit zusätzlichen Operationen verbunden, wobei krankhafte und schlecht heilende Wunden entstehen können und die Einheilung verzögert wird.

Die Schwierigkeiten, die mit der Stärkung der Hornhaut mittels Auto- bzw. Alloimplantaten verbunden sind, lenken die Aufmerksamkeit auf polymere Materialien.

Bekannt ist ein Implantat aus porösem, expandiertem Polytetrafluorethylen, das im Kaninchenexperiment untersucht wurde (Legeasis Z-M. et al., Exp. Eye Res., 1994, V. 58, S. 41-52). Poröses, expandiertes Polytetrafluorethylen (PTFE) hat eine Struktur aus Polymerknoten, die durch 7-8 µm lange Fibrillen verbunden sind. Zusätzlich wird das Material mit vertikalen und zur Folienoberfläche senkrechten Poren mit einem Durchmesser von 20 µm und 50 µm durchdrungen. Die Gesamtporösität des Implantats beträgt 44±5 % für Porendurchmesser von 20 µm und 88±4 % für Porendurchmesser von 50 µm. Die hinsichtlich der Dicke gleichartigen Scheiben sind 0,2 mm (200 µm) dick und haben einen Durchmesser von 6 mm. Die Erfinder beobachteten eine Augenimplantatverwachsung mit Stromazellen, wobei sich das Ergebnis für Poren mit einem Durchmesser von 50 µm als besser erwies. Ohne zusätzliche Poren verwächst poröses, expandiertes PTFE, das eine Knoten-Fibrillen-Struktur hat, mit Stromazellen nicht, und das PTFE wird von der Hornhaut abgestoßen.

Ein Implantat aus porösem, expandiertem PTFE mit zusätzlichen, vertikalen Poren von 20-150 µm, vorzugsweise 50 µm, im Durchmesser wird zum Einsatz als Tragplatte einer Keratoprothese im US-Patent 5 713 956, IPC A61F2/14, veröffentlicht am 03.02.1998, vorgeschlagen. Die Dicke der Tragplatte gemäß diesem Patent beträgt 0,2-0,3 mm. Ein derartig dickes Material wird transparent, wobei es mit dem Hornhautgewebe verwächst. Eine Platte mit einer Dicke über 0,3mm wird nicht transparent.

Polytetrafluorethylen besitzt eine hohe chemische Passivität und ist gegen eine biologische sowohl zelluläre als auch nicht zelluläre Zerstörung beständig. Seine thermische Beständigkeit (zerfällt bei einer Temperatur von über 350-380° C) lässt eine thermische Sterilisation zu. Poröses, expandiertes PTFE wird mittels Orientierung einer extrudierten Folie, die aus Emulsionspolytetrafluorethylen gewonnen wird, beispielsweise aus Fluorplast-4D, hergestellt. Um ein Material zur Hornhautstärkung zu erhalten, soll poröses, expandiertes PTFE mit vertikalen Poren von 20µm bzw. 50µm im Durchmesser durchdrungen werden; dies bildet jedoch ein technisch kompliziertes und kostspieliges Verfahren, weil jede Pore einzeln mit dem Laser oder einer anderen Verfahrenstechnik hergestellt werden muss.

Bekannt ist eine Keratoprothese, die aus einem optischen Element aus transparentem Stoff, beispielsweise Polymethylmethakrylat (PMMA), und einer Ringtragplatte aus hydrophilem, porösen Material besteht, das zum Verwachsen mit Hornhautgewebe fähig ist (US-Patent 5 489 301, IPC 7A61 F2/14, veröffentlicht am 05.02.1996).

Hydrophiles, poröses Material besteht aus PTFE, das mit Ruß oder Aluminiumoxid, vorzugsweise mit Letzterem in Form von Fasern, gefüllt ist. Aus den Fasern wird eine Platte von 0,15-0,30 µm Dicke ausgebildet. Der Ring mit einem Außendurchmesser von 9,5 mm und mehr wird auf das optische Element gesetzt, dessen Vorderseite zylindrisch und als Kegelstumpf mit einem dem Augeninnern zugekehrter Teil ausgebildet ist, dessen größerer Schnitt zum Augeninnern weist. Die Ringtragplatte hat ausreichende Elastizitätsparameter, um einer Abstoßung der Keratoprothese und einer Nekrose infolge des Drucks des umgebenden Augengewebes entgegenzuwirken, wenn die Prothese im Auge implantiert ist.

Keratoprothesen derartiger Bauweise wurden in Augen von 11 Katzen implantiert, wobei die Tragplatte im intralamellären Hornhautkanal angeordnet wurde. 9 Keratoprothesen blieben 28 Wochen lang zur Untersuchung im Auge. 2 Prothesen wurden infolge einer Entzündung und einer Infektion abgestoßen.

Ein Nachteil dieser Keratoprothese ist, dass das konische Hinterteil des optischen Elements die Implantation erschwert und die Wunde in diesem Fall nicht zuverlässig abgedichtet wird. Eine schlechte Dichtheit kann zur Infektion und Abstoßung der Prothese führen. Ferner lässt das bekannte Herstellungsverfahren der Platten aus PTFE-Fasern eine Wiederherstellung ihrer Struktur und Porosität nicht zu. Ein Material mit vorgegebener Struktur kann nicht gewonnen werden.

Bekannt ist eine Keratoprothese, die ein optisches Element aus optisch transparentem Material, beispielsweise aus Polymethylmethacrylat, und eine Tragplatte aus porösem Polytetrafluorethylen oder porösem Polyethylen umfasst (US-Patent 6 106 552, IPC A61F2/14, veröffentlicht am 22.08.2000). Die Tragplatte der Prothese wird in Form von zwei parallelen Umhüllungen ausgeführt, die im Abstand von 0,4-0,7 µm, vorzugsweise im Abstand von 0,3±0,02 µm, voneinander angeordnet, leicht konisch sind und an das zylindrische, optische Element befestigt werden.

Damit das Material der Tragplatte biologisch "besiedelt" wird, soll diese aus Material mit einer Porosität von 50 % und mehr hergestellt werden. Der Porendurchmesser soll etwa 20-100 µm betragen, und die Poren selbst sollen offen sein. Daten über Prüfungen derartiger Keratoprothesen sind zurzeit nicht verfügbar.

Die Untersuchungen zeigten, dass dünne Platten aus Polytetrafluorethylen mit einer Porosität von 50 % und mehr über eine unzureichende, mechanische Festigkeit verfügen, als dass sie zur Herstellung von Keratoprothesen verwendet werden können. Darüber hinaus erhöht ein großer Durchmesser des optischen Elements in Verbindung mit einem kleinen Durchmesser der Tragplatte und deren unzureichenden Festigkeit das Abstoßungsrisiko der Keratoprothese unter der Wirkung des Augeninnendrucks, der Augenflüssigkeitsfiltration und der Infektionseindringung in das Augeninnere.

Die Aufgabe der Erfindung besteht darin, eine Keratoprothese zu schaffen, mit der die Einheilung des Implantats in der Augenhornhaut verbessert ist.

Der Erfindung ist auch die Aufgabe zugrunde gelegt, das Abstoßungsrisiko von Keratoprothesen zu minimieren und die Einheilung der Tragplatten dieser Keratoprothesen zu erhöhen.

Eine weitere Aufgabe der Erfindung besteht darin, ein gut in der Augenhornhaut einheilendes, einfaches Material kostengünstig herzustellen.

Das Implantat zur Hornhautstärkung gemäß der vorliegenden Erfindung wird aus porösem PTFE hergestellt, das über eine Struktur aus einem Polymer und Hohlräumen mit deren Verbindung in ein dreidimensionales Netz verfügt, das einen Hohlraumanteil von 15-40 %, eine spezifische Hohlraumoberfläche von 0,25-0,55 µm²/µm³, einen durchschnittlichen Abstand zwischen den Hohlräumen von 25-50 µm und eine durchschnittliche Strangstärke von 8-25 µm aufweist.

Das Implantat kann die Form eines Kreises, eines Ovals, eines Dreiblatts, einer Kamille oder einer Konvexkonkavlinse, vorzugsweise letzterer, aufweisen. Die Linse weist einen dem menschlichen Auge entsprechenden Krümmungsradius von 7-10 mm auf. Das Implantat kann in der Mitte eine zylindrische Öffnung zur Aufnahme einer Keratoprothese haben.

Erfindungsgemäß umfasst die Keratoprothese ein optisches Element aus optisch transparentem Material und eine Tragplatte aus porösem PTFE. Dieses verfügt über eine Struktur aus Polymer und Hohlräumen mit deren Verbindung in ein dreidimensionales Netz, das einen Hohlraumanteil von 15-40%, eine spezifische Hohlraumoberfläche von 0,25-0,55 µm²/µm³, einen durchschnittlichen Abstand zwischen den Hohlräumen von 25-50 µm und eine durchschnittliche Strangstärke (Chorda) von 8-25 µm aufweist.

Die Tragplatte der Keratoprothese kann als Konvexkonkavlinse mit einem Krümmungsradius von 7-10 mm und in der Mitte mit einer zylindrischen Öffnung ausgebildet werden.

Das optische Element kann als Zylinder aus optisch transparentem Material, wie Polymethylmethacrylat oder Polycarbonat, ausgebildet werden. Das optische Element kann zerlegbar sein.

Die Gewinnung von PTFE für das Implantat und die Tragplatte der Keratoprothese wird unten beschrieben.

Suspensionspolytetrafluorethylen, beispielsweise Fluorplast-D4 (F-4, GOST-Norm 10007-80), wird in Form von Pulver auf eine konstante Temperatur von 380±10° C gebracht und zusätzlich zerkleinert. Das Pulver wird in Fraktionen von 0,25-1,60 mm gesiebt. Aus der gesiebten Fraktion wird ein Werkstück unter einem Druck von 30±5 MPa gepresst und 5-6 Stunden lang bei einer Temperatur von 380±10° C gesintert.

Eine Folie mit vorgegebener Dicke entsteht durch Abhobeln. Die Folie lässt sich gut mit Scheren schneiden, und wird so auf die benötigte Form gebracht.

Die stereologischen (volumetrischen) Parameter der Tragplatte werden nach einem bekannten Verfahren bestimmt (W. G. Pantelejew, K. S. Ramm, Nichtorganische Materialien, 1986, Band 22, Nr. 12, S. 1941-1951; G. G. Awtangilow et al., Systemstereometrie bei Untersuchung eines pathologischen Prozesses, Moskau, Verlag Medizin, 1981; K. S. Tschernjawskij, Stereologie in Metallkunde, Moskau, Verlag Metallurgie, 1977).

Gemäß der Erfindung weist die Materialstruktur der Tragpatte einen Hohlraumanteil von 15-40 %, eine spezifische Hohlraumoberfläche von 0,25-0,55 µm²/µm³, einen durchschnittlichen Abstand zwischen den Hohlräumen von 25-50 µm und eine durchschnittliche Strangstärke von 8-25 µm auf.

Die Erfindung wird nun anhand eines Ausführungsbeispiel und den beigefügten Zeichnungen näher erläutert. Es zeigen:
- Fig. 1: ein Oberflächenbild von porösem Polytetrafluorethylen, aus dem ein Implantat besteht,
- Fig. 2a: einen Schnitt durch ein Implantat zur Stärkung der Augenhornhaut,
- Fig. 2b: eine Draufsicht auf das Implantat der Fig. 2a,
- Fig. 3: einen Schnitt durch eine Keratoprothese mit einem nicht zerlegbaren, optischen Element,
- Fig. 4: einen Schnitt durch eine Keratoprothese mit einem zerlegbaren, optischen Element,
- Fig. 5: einen Schnitt bzw eine Seitenansicht eines zerlegbaren, optischen Elements der Keratoprothese,
- Fig. 6a: einen Schnitt bzw eine Seitenansicht eines zerlegbaren, optischen Elements der Keratoprothese,
- Fig. 6b: einen Schnitt bzw eine Seitenansicht einer zur Keratoprothese der Fig. 6a zugehörigen Scheibe,
- Fig. 7: einen Schnitt durch einen Teil der Augenhornhaut mit der in die Augenhornhaut implantierten Keratoprothese zur Darstellung der Lage der Keratoprothese,
- Figuren 8-11: Mikroaufnahmen eines Implantats, das mit dem Hornhautgewebe verwachsen ist.

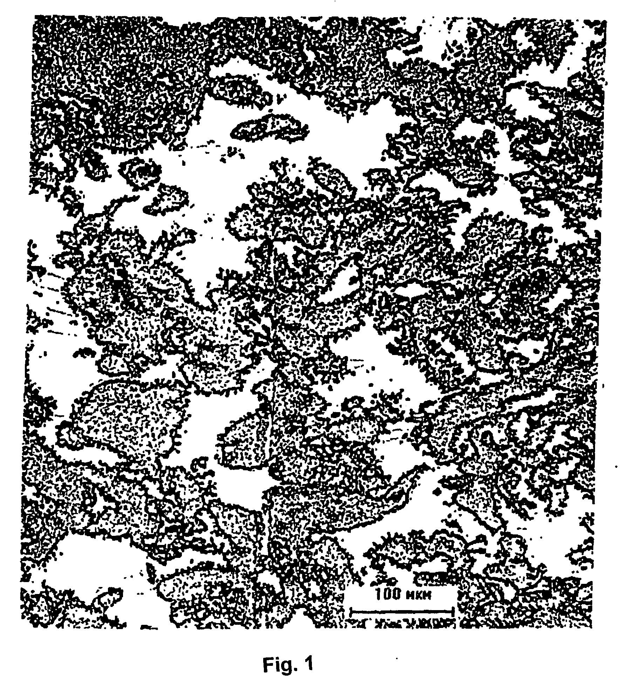

Aus der Mikroaufnahme von porösem Polytetrafluoräthylen in Fig. 1, das zur Herstellung des Implantats und der Tragplatte der Keratoprothese eingesetzt wird, sind ein dreidimensionales Netz, das durch Hohlräume ausgebildet ist, und Knoten der Polymermatrix klar erkennbar.

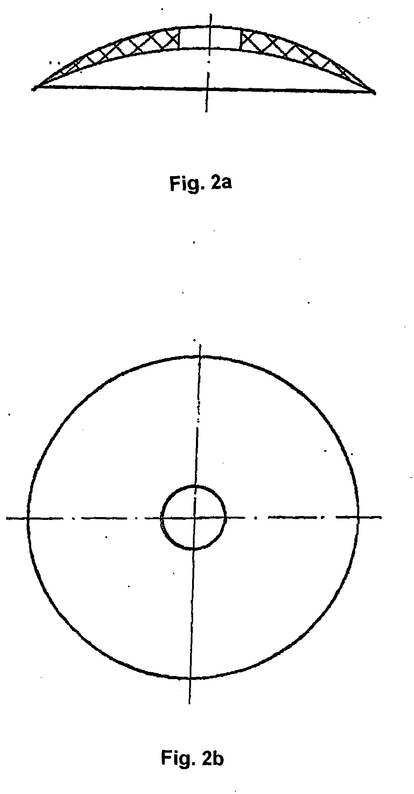

Das Implantat gemäß der Erfindung (Figuren 2a, 2b) stellt eine Konvexkonkavlinse (Fig. 2a) dar, die einen der Hornhautkrümmung des Kranken entsprechenden Krümmungsradius aufweist. Im mittleren Teil ist das Implantat 0,3-0,7 mm und an den Enden bis 0,01 mm dick. In der Mitte weist das Implantat eine zylindrische Öffnung (Fig. 2b) zur Aufnahme der Keratoprothese auf.

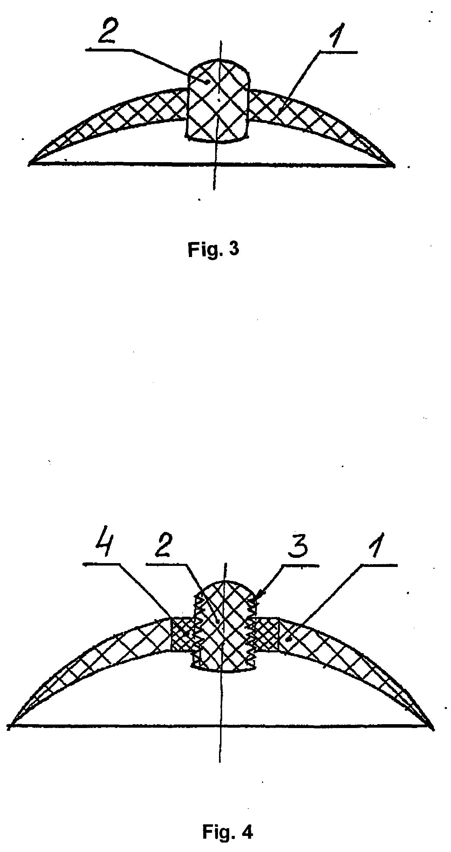

Die Keratoprothese (Figuren 3, 4) umfasst eine Tragplatte 1 und ein optisches Element 2. Die Tragplatte 1 ist als Konvexkonkavlinse mit einem Krümmungsradius von 7-10 mm wie das Implantat zur Hornhautstärkung in den Figuren 2a, 2b ausgeführt. Im konkreten Fall entspricht der Krümmungsradius der Tragplatte dem Hornhautkrümmungsradius des Kranken. Der Krümmungsradius wird mittels harter Kontaktlinsen oder mit einem anderen Verfahren festgelegt. Der an das optische Element 2 anliegende Abschnitt der Tragplatte 1 ist 0,3-0,7 mm dick, an den Enden ist die Tragplatte 0,01 mm dick. Der Außendurchmesser der Tragplatte 1 kann 7-12 mm in Abhängigkeit vom Hornhautdurchmesser des Kranken betragen. In der Mitte weist die Tragplatte 1 eine zylindrische Öffnung zur Aufnahme des optischen Elementes 2 auf.

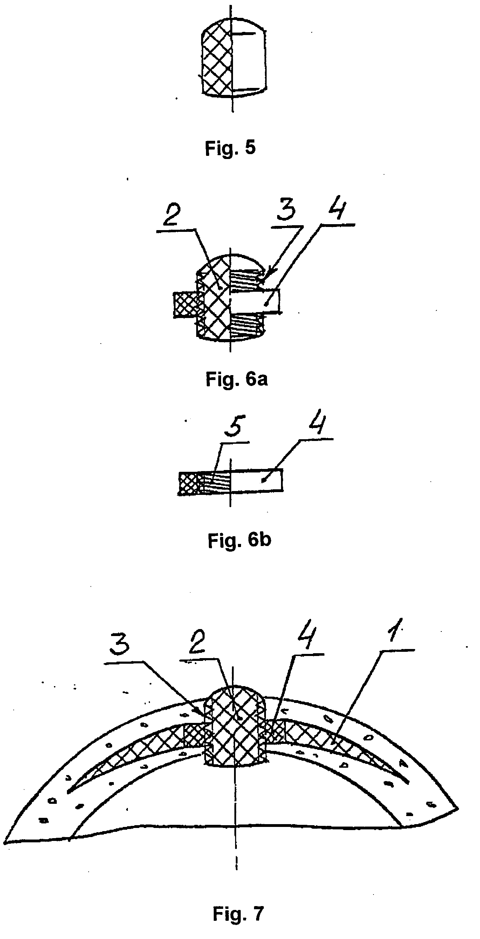

Das optische Element 2 kann als Zylinder (Figuren 3, 5) ausgeführt werden. Die äußere Stirnseite (entgegen der Richtung zum Auge) des Zylinders ist immer konvex. Die innere Stirnseite kann in Abhängigkeit von der Dioptrienzahl des optischen Elements eben oder leicht gekrümmt sein. Das optische Element wird aus Polymethylmethakrylat oder Polykarbonat ausgeführt. Die optischen Elemente können mit 40-60 Dioptrien ausgeführt werden.

Wenn das optische Element der Keratoprothese gemäß der Erfindung zerlegbar ausgeführt wird (Figuren 4, 6a), ist auf der Seitenoberfläche des Zylinders ein Gewinde 3 eingeschnitten und die Prothese mit einer zusätzlichen Scheibe 4 versehen, die ein Innengewinde 5 (Fig. 6b) aufweist. Die Scheibe 4 ist mit ihrer Außenseite mit der Innenfläche der zylindrischen Öffnung in der Tragplatte 1 verbunden bzw. zusammengeklebt.

Die Keratoprothese kann in eine intralamelläre Hornhauttasche, wie sie in Fig. 7 dargestellt ist, implantiert werden. Die Tragplatte 1 ist dabei in der Augenhornhaut angeordnet, und das optische Element 2 geht durch alle Hornhautschichten hindurch.

Das Implantat wurde bei Kaninchen der Rasse Chinchilla mit einem Gewicht von 3,7-4,3 kg auf das Anheilungsvermögen geprüft. Unter allgemeiner Anästhesie und Tropfanästhesie wurde die Hornhaut auf einer Länge von 5 mm und 4/5 der Hornhaut tief im Limbus cornea konzentrisch eingeschnitten. In dieser Fläche wurde eine intralamelläre Tasche ausgeformt, in die das Implantat eingeführt wurde. Die Hornhaut wurde nicht genäht. Bei den Tieren wurde nur ein Auge operiert, um ihre Orientierungsfähigkeit in der Umgebung zu erhalten.

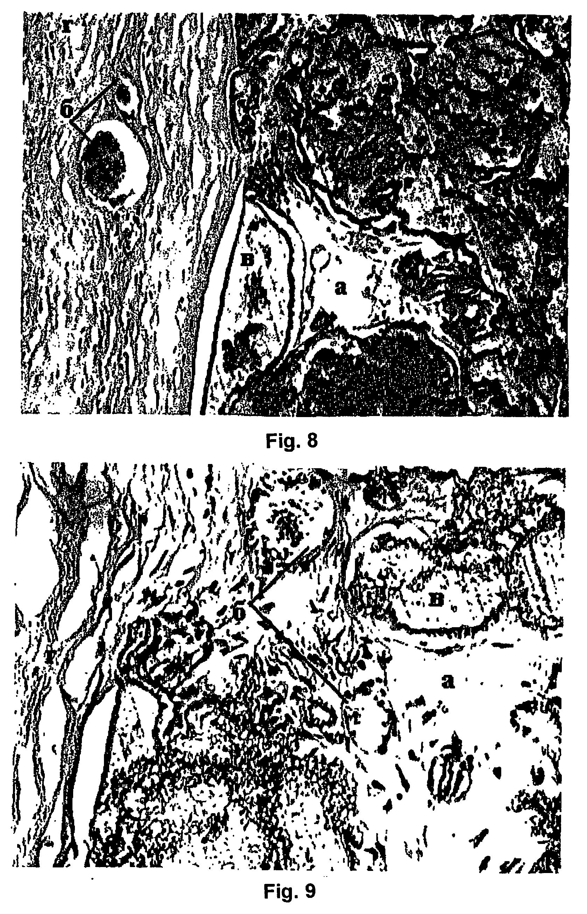

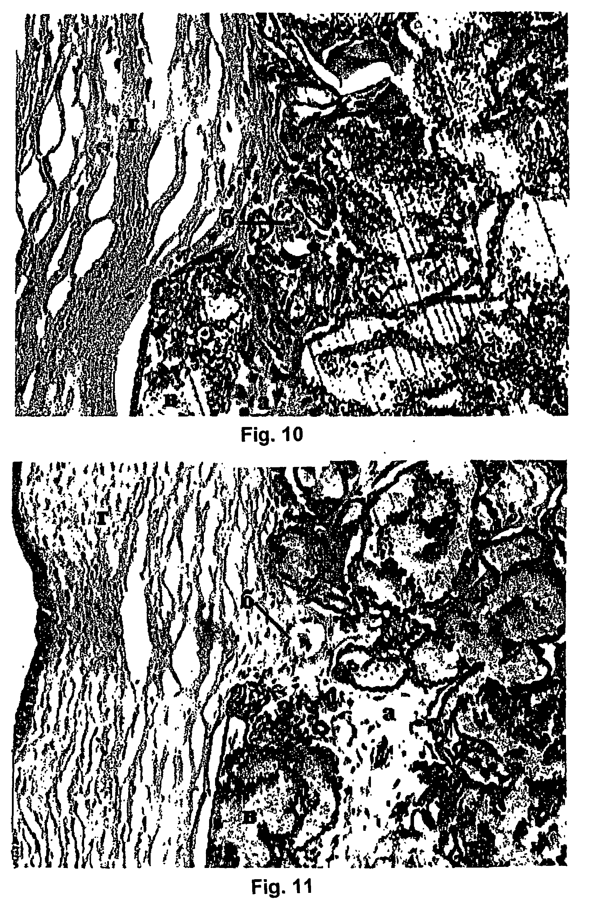

Nach 4 Monaten wurden die Präparate durch Kernbohren der ganzen Hornhaut histologisch untersucht. Die Untersuchungsergebnisse sind in den Figuren 8-11 dargestellt. Wie aus den Mikroaufnahmen von Querschnitten der gestärkten Hornhaut ersichtlich ist, wachsen das Bindegewebe (a) und einzelne Blutgefäße (b) in Implantathohlräume zwischen den Polymerelementen (B) ein. Der Hornhautabschnitt (r) über dem Implantat und in anderen Abschnitten blieb transparent sowie ohne Hypoxieanzeichen und Trophik im Stromagewebe, in der Bowmanschen Kapsel und im Ephitel. Diese Ergebnisse weisen eine fehlende Barriere des Implantats gemäß der Erfindung zur Diffusion von Nährstoffen und Sauerstoff in das Hornhautgewebe nach. Es wurden keine Implantatabstoßungen im Verlauf von 6 Monaten beobachtet.

Eine gute Einheilung des porösen PTFE mit der genannten Struktur ermöglicht es, das Leukom auf die Keratoprothetik vorzubereiten. Unter das eingeheilte Implantat oder im Implantatinnern kann eine Keratoprothese jeder bekannten Ausbildungsart angeordnet sein. Wenn die Tragplatte der Keratoprothese aus dem genannten PTFE hergestellt wird und die Hornhaut für die Implantation ausreichend dick ist, besteht keine Notwendigkeit, die Hornhaut zu stärken, und das Abstoßungsrisiko ist wesentlich geringer.

Das Implantat gemäß der Erfindung und die Keratoprothese können in Unternehmen hergestellt werden, die Polymere für medizinische Zwecke und medizinische Optik erzeugen. Sie können ihre Anwendung in der ophtalmologischen Chirurgie bei Leukomoperationen finden.

## Patentansprüche

1. Implantat zur Hornhautstärkung, das aus porösem Polytetrafluorethylen besteht,
**dadurch gekennzeichnet,**
**dass** das poröse Polytetrafluorethylen über eine Struktur aus einem Polymer und Hohlräumen mit Verbindungen dieser Hohlräume in ein dreidimensionales Netz verfügt, das einen Hohlraumanteil von 15-40 %, eine spezifische Holraumoberfläche von 0,25-0,55 µm2/µm3, einen Durchschnittsabstand zwischen den Hohlräumen von 25-50 µm und eine durchschnittliche Strangstärke von 8-25 µm aufweist.

2. Implantat nach Anspruch 1,
**dadurch gekennzeichnet,**
**dass** es die Form einer Konvexkonkavlinse mit einem Krümmungsradius von 7,0-10,0 mm aufweist.

3. Implantat nach Anspruch 2,
**dadurch gekennzeichnet,**
**dass** die Linse in der Mitte eine zylindrische Öffnung aufweist.

4. Keratoprothese, die ein optisches Element aus optisch transparentem Material und eine mit diesem Element verbundene Tragplatte aus porösem Polytetrafluorethylen umfasst,
**dadurch gekennzeichnet,**
**dass** das poröse Polytetrafluotethylen über eine Struktur aus einem Polymer und Hohlräumen mit Verbindung dieser Hohlräume in ein dreidimensionales Netz verfügt, das einen Hohlraumanteil von 15-40 %, eine spezifische Holraumoberfläche von 0,25-0,55 µm2/µm3, einen Durchschnittsabstand zwischen den Hohlräumen von 25-50 µm und eine durchschnittliche Strangstärke von 8-25 µm aufweist.

5. Keratoprothese nach Anspruch 4,
**dadurch gekennzeichnet,**
**dass** die Tragplatte eine konvexkonkave Form mit einem Krümmungsradius von 7-10 mm und in der Mitte eine zylindrische Öffnung aufweist.

6. Keratoprothese nach Anspruch 4,
**dadurch gekennzeichnet,**
**dass** das optische Element aus Polymethylmethakrylat besteht.

7. Keratoprothese nach Anspruch 4,
**dadurch gekennzeichnet,**
**dass** das optische Element aus Polykarbonat besteht.

8. Keratoprothese nach Anspruch 4,
**dadurch gekennzeichnet,**
**dass** das optische Element zerlegbar ausgebildet ist.

9. Keratoprothese nach Anspruch 4,
**dadurch gekennzeichnet,**
**dass** die Seitenfläche des optischen Elements mit einem Gewinde versehen ist und dass das optische Element eine zusätzliche Scheibe umfasst, die ein Innengewinde aufweist und mit der Tragplatte verbunden ist.
